# EUROPEAN PATENT APPLICATION

(11) **EP 1 356 788 A2**
(43) Date of publication of application: **29.10.2003**
(21) Application number: 03250108.2
(22) Date of filing: 08.01.2003
(51) Int. Cl.: A61F 2/06

(54) **Modular aneurysm repair system**

(30) Priority: 08.01.2002 US 41117
(71) Applicant: Cordis Corporation, Miami Lakes Florida 33014 (US)
(72) Inventor: Depalma, Donald F., Weston, FL 33326 (US); Dwyer, Clifford J., Weston, FL 33326 (US); Letendre, Robert P., Hialeah, FL 33015 (US)
(74) Representative: Belcher, Simon James

(57) **Abstract**

A system for bypassing an aneurysm (100) comprises a first prosthesis (10) and at least one second prosthesis (11a,11b) communicating with the first prosthesis (10). The first prosthesis (10) comprises a conduit defining a fluid flow path; and the second prosthesis (11a,11b) is configured to provide a fluid flow path through the aneurysm (100).

## Description

The present invention relates to devices for repairing aneurysms, and more particularly, to intraluminally and/or percutaneously delivered devices for repairing aneurysms, such as abdominal aortic aneurysms and thoracic aortic aneurysms.

An aneurysm is an abnormal dilation of a layer or layers of an arterial wall, usually caused by a systemic collagen synthetic or structural defect. An abdominal aortic aneurysm is an aneurysm in the abdominal portion of the aorta, usually located in or near one or both of the two iliac arteries or near the renal arteries. The aneurysm often arises in the infrarenal portion of the diseased aorta, for example, below the kidneys. A thoracic aortic aneurysm is an aneurysm in the thoracic portion of the aorta. When left untreated, the aneurysm may rupture, usually causing rapid fatal haemorrhaging.

Aneurysms may be classified or typed by their position as well as by the number of aneurysms in a cluster. Typically, abdominal aortic aneurysms may be classified into five types. A Type I aneurysm is a single dilation located between the renal arteries and the iliac arteries. Typically, in a Type I aneurysm, the aorta is healthy between the renal arteries and the aneurysm and between the aneurysm and the iliac arteries.

A Type II A aneurysm is a single dilation located between the renal arteries and the iliac arteries. In a Type II A aneurysm, the aorta is healthy between the renal arteries and the aneurysm, but not healthy between the aneurysm and the iliac arteries. In other words, the dilation extends to the aortic bifurcation. A Type II B aneurysm comprises three dilations. One dilation is located between the renal arteries and the iliac arteries. Like a Type II A aneurysm, the aorta is healthy between the aneurysm and the renal arteries, but not healthy between the aneurysm and the iliac arteries. The other two dilations are located in the iliac arteries between the aortic bifurcation and the bifurcations between the external iliacs and the internal iliacs. The iliac arteries are healthy between the iliac bifurcation and the aneurysms. A Type II C aneurysm also comprises three dilations. However, in a Type II C aneurysm, the dilations in the iliac arteries extend to the iliac bifurcation.

A Type III aneurysm is a single dilation located between the renal arteries and the iliac arteries. In a Type III aneurysm, the aorta is not healthy between the renal arteries and the aneurysm. In other words, the dilation extends to the renal arteries.

A ruptured abdominal aortic aneurysm is presently the thirteenth leading cause of death in the United States. The routine management of abdominal aortic aneurysms has been surgical bypass, with the placement of a graft in the involved or dilated segment. Although resection with a synthetic graft via transperitoneal or retroperitoneal approach has been the standard treatment, it is associated with significant risk. For example, complications include perioperative myocardial ischemia, renal failure, erectile impotence, intestinal ischemia, infection, lower limb ischemia, spinal cord injury with paralysis, aortaenteric fistula, and death. Surgical treatment of abdominal aortic aneurysms is associated with an overall mortality rate of five percent in asymptomatic patients, sixteen to nineteen percent in symptomatic patients, and is as high as fifty percent in patients with ruptured abdominal aortic aneurysms.

Disadvantages associated with conventional surgery, in additional to the high mortality rate, include an extended recovery period associated with the large surgical incision and the opening of the abdominal cavity, difficulties in suturing the graft to the aorta, the loss of the existing thrombosis to support and reinforce the graft, the unsuitability of the surgery for many patients having abdominal aortic aneurysms, and the problems associated with performing the surgery on an emergency basis after the aneurysm has ruptured. Further, the typical recovery period is from one to two weeks in the hospital, and a convalescence period at home from two to three months or more, if complications ensue. Since many patients having abdominal aortic aneurysms have other chronic illnesses, such as heart, lung, liver and/or kidney disease, coupled with the fact that many of these patients are older, they are less than ideal candidates for surgery.

The occurrence of aneurysms is not confined to the abdominal region. While abdominal aortic aneurysms are generally the most common, aneurysms in other regions of the aorta or one of its branches are possible. For example, aneurysms may occur in the thoracic aorta. As is the case with abdominal aortic aneurysms, the widely accepted approach to treating an aneurysm in the thoracic aorta is surgical repair, involving replacing the aneurysmal segment with a prosthetic device. This surgery, as described above, is a major undertaking, with associated high risks and with significant mortality and morbidity.

Over the past five years, there has been a great deal of research directed at developing less invasive, percutaneous, e.g., catheter directed, techniques for the treatment of aneurysms, specifically abdominal aortic aneurysms. This has been facilitated by the development of vascular stents, which can and have been used in conjunction with standard or thin-wall graft material in order to create a stent-graft or endograft. The potential advantages of less invasive treatments have included reduced surgical morbidity and mortality along with shorter hospital and intensive care unit stays.

Stent-grafts or endoprostheses are now FDA approved and commercially available. The delivery procedure typically involves advanced angiographic techniques performed through vascular accesses gained via surgical cutdown of a remote artery, such as the common femoral or brachial arteries. Over a guidewire, the appropriate size introducer will be placed. The catheter and guidewire are passed through the aneurysm. Through the introducer, the stent-graft will be advanced to the appropriate position. Typical deployment of the stent-graft device requires withdrawal of an outer sheath while maintaining the position of the stent-graft with an inner-stabilizing device. Most stent-grafts are self-expanding; however, an additional angioplasty procedure, e.g., balloon angioplasty, may be required to secure the position of the stent-graft. Following the placement of the stent-graft, standard angiographic views may be obtained.

Due to the large diameter of the above-described devices, typically greater than twenty French (3F = 1 mm), arteriotomy closure requires surgical repair. Some procedures may require additional surgical techniques, such as hypogastric artery embolisation, vessel ligation, or surgical bypass, in order to adequately treat the aneurysm or to maintain flow to both lower extremities. Likewise, some procedures will require additional, advanced catheter directed techniques, such as angioplasty, stent placement, and embolisation, in order to successfully exclude the aneurysm and efficiently manage leaks.

While the above-described endoprostheses represent a significant improvement over conventional surgical techniques, there is a need to improve the endoprostheses, their method of use and their applicability to varied biological conditions. Accordingly, in order to provide a safe and effective alternate means for treating aneurysms, including abdominal aortic aneurysms and thoracic aortic aneurysms, a number of difficulties associated with currently known endoprostheses and their delivery systems must be overcome. One concern with the use of endoprostheses is the prevention of endo-leaks and the disruption of the normal fluid dynamics of the vasculature. Devices using any technology should preferably be simple to position and reposition as necessary, should preferably provide an acute fluid tight seal, and should preferably be anchored to prevent migration without interfering with normal blood flow in both the aneurysmal vessel as well as branching vessels. In addition, devices using the technology should preferably be able to be anchored, sealed, and maintained in bifurcated vessels, tortuous vessels, highly angulated vessels, partially diseased vessels, calcified vessels, odd shaped vessels, short vessels, and long vessels. In order to accomplish this, the endoprostheses should preferably be extendable and re-configurable while maintaining acute and long term fluid tight seals and anchoring positions.

The endoprostheses should also preferably be able to be delivered percutaneously utilizing catheters, guidewires and other devices which substantially eliminate the need for open surgical intervention. Accordingly, the diameter of the endoprostheses in the catheter is an important factor. This is especially true for aneurysms in the larger vessels, such as the thoracic aorta.

The modular aneurysm repair system of the present invention provides a means for overcoming the problems associated with anchoring and/or sealing a by-pass prosthesis in a highly angulated arterial section, a too short section of artery, a diseased section of artery or at the junction of arterial branches as briefly described above.

The modular aneurysm repair system of the present invention provides a means for overcoming the problems associated with anchoring and/or sealing a by-pass prosthesis in a highly angulated arterial section, a too short section of artery, a diseased section of artery or at the junction of arterial branches as briefly described above.

As will be recognized by those skilled in the art, placing a prosthesis upstream of an aneurysm requires a sufficient length of suitable artery within which to anchor an upstream portion of the prosthesis. For some patients, a suitable length of artery upstream of the aneurysm is not available. For example, a Schumacher Type III abdominal aortic aneurysm is typically characterized by a short infra-renal neck (i.e., the section of the artery downstream of the renal arteries and upstream of an aneurysm is typically less than about fifteen mm) and/or a high angulated neck (greater than about forth-five degrees). In both of these circumstances, it is typically not possible to implant a prosthesis upstream of the aneurysm without blocking one or both of the renal arteries. Also, the shape, angle, or length of the existing artery may prevent achieving a fluid tight connection between the prosthesis and the arterial wall.

Therefore, a need exists for a prosthesis specifically designed to accommodate a short section of artery, to accommodate a section of artery that includes an arterial junction, and/or to accommodate a highly angulated section of artery. A need also exists for a modular system specifically configured to all types of abdominal aortic aneurysms.

Finally, it may be desirable to provide a system in which the first prosthesis is both a seal for the system, and a seat for one or more additional prostheses that provide one or more fluid flow paths. In some embodiments of the invention, e.g., those in which the system is positioned upstream of a cross artery, the first prosthesis also provides at least one additional fluid flow path into the cross-artery.

Prior systems typically provide a first prosthesis that anchors the system in the artery and anchors one or more other prostheses that establish a fluid flow path through the system. In these systems, the anchoring prosthesis typically does not itself conduct fluid, such as blood. In accordance with the present invention, it has been found that the treatment of some aneurysms may benefit by providing a first prosthesis that anchors the system, anchors one or more other prostheses, and provides at least one fluid flow path through the system.

The present invention is directed to a system including at least one prosthesis for repair or replacement of a mammalian body part or condition. The typical system includes a first prosthesis for sealing the system within a predetermined portion of an artery; at least one second prosthesis engaged to the first prosthesis, said second prosthesis providing a fluid flow path through the system or a portion of the system; and a third or extension prosthesis for extending a fluid flow path through the system or a portion of the system. In some embodiments of the invention, the second prosthesis is sealingly and/or matingly engaged with the first prosthesis. In some embodiments of the invention, the extension prosthesis extends the fluid flow path formed by the second prosthesis. In some embodiments of the invention, the extension prosthesis is sealingly and/or matingly engaged with the second prosthesis. The first prosthesis is further adapted to receive at least one second prosthesis, and is also configured to provide a fluid flow path, preferably from a proximal end of the system into one or more second prostheses.

A typical first prosthesis includes a support or stent structure, and a foam or gasket material supported by the stent, the stent and gasket material being configured to seal the system within an artery. A typical first prosthesis also includes one or more structures or elements for engaging the second prosthesis. In preferred embodiments of the invention, these elements or structures sealing and/or matingly engage the second prosthesis. The stent is typically a synthetic or natural matrix for supporting the gasket material. In some exemplary embodiments of the stent, the stent is a hollow, substantially cylindrical, and preferably radially expandable matrix having a lumen and two open ends. The typical gasket material is a synthetic or natural fabric, tissue, foam, or the like. In preferred embodiments of the invention, the gasket material covers at least a portion of the lumen, even more preferably, the proximal end of the lumen.

In accordance with the present invention, the predetermined position, as used herein, refers to a section of artery upstream of an aneurysm, the section being unsuitable for anchoring a prosthesis.

In accordance with the present invention, a section is unsuitable if it is non-existent, too short, too bent or angulated, includes another artery (typically, a cross-flow artery), or any other condition, for example, calcified sections, in which it would be desirable or beneficial to anchor the prosthesis upstream of the unsuitable section of artery. A section is also unsuitable if it would be deleterious to place a fluid tight prosthesis within a section of artery in which continued blood flow is desirable.

Previous configurations of an anchoring prosthesis are relatively inflexible, and have a proximal end designed to engage a proximal end of a second prosthesis. In these systems, the anchoring prosthesis itself is not configured to provide a fluid flow path through the anchoring prosthesis; it acts as an anchor for positioning other prostheses that are configured as a fluid flow path.

In the present invention, the sealing prosthesis itself is configured not to form a fluid flow path. Further, the sealing prosthesis comprises a manifold, the manifold being configured to engage at least one second prosthesis, the second prosthesis defining a fluid flow path. Further, the second prosthesis, when fully deployed or expanded, provides radial force against the walls of the artery in order to anchor the system in the artery. In preferred embodiments of the invention, the manifold is configured to engage a second prosthesis defining a second fluid flow path, and a third prosthesis defining a third fluid flow path. In most preferred embodiments of the invention, the anchoring prosthesis may be adapted into the shape of the artery. For example, if the artery has an "S" shape, the preferred anchoring prosthesis is flexible enough to assume an "S" shape that conforms to the shape of the artery.

The present invention is directed to a system for bypassing an aneurysm comprising a first prosthesis defining a lumen that may or may not be a fluid flow path; a manifold positioned within a distal portion of the first prosthesis; and at least one second prosthesis adapted to engage the manifold, the second prosthesis defining a second fluid flow path. In preferred embodiments of the invention, the second prosthesis engages a portion of the manifold. In the most preferred embodiments of the invention, the manifold is configured to engage a second and a third prosthesis, each of the second and third prostheses defining separate fluid flow paths.

The typical second prosthesis of the present invention includes a support or stent structure, and graft material supported by the stent, the stent and graft material defining a fluid flow path therethrough. The typical graft material is a synthetic or natural fabric, tissue, or the like. The stent is typically a synthetic or natural matrix for supporting the graft and/or positioning the prosthesis in a pre-determined position. In some embodiments of the stent, the stent is a hollow, substantially cylindrical, and preferably radially expandable matrix having a lumen and two open ends. The stent typically comprises a plurality of interconnected struts. In some embodiments of the invention, a graft material may be positioned on an inside and/or outside surface of the matrix; in preferred embodiments of the invention, the graft material may include a plurality of substantially longitudinally directed pleats disposed thereon. In a particularly preferred embodiment, the graft further includes a plurality of radially oriented pleat interruptions. In some embodiments of the invention the graft material may be attached to the stent, preferably by one or more staples or the like.

The typical first prosthesis of the present invention also includes a manifold or the like configured to engage and receive at least one second prosthesis. The manifold is typically positioned on or near the distal or downstream end of the first prosthesis, across the fluid flow path. The typical manifold is formed of the same material used to form the gasket material, e.g., a synthetic or natural fabric, tissue, or the like.

In the embodiments of the invention in which the first prosthesis is suitable for placement upstream of, or across an arterial junction, the first prosthesis may be further configured with a second manifold positioned in or near a proximal end of the first prosthesis, preferably upstream of the first manifold and upstream of the cross artery. In a preferred embodiment of the invention, the second manifold has all the attributes and functions of the first manifold, and is adapted to receive at least one third prosthesis for establishing a fluid flow path into a cross artery.

A prosthesis according to the present invention is specifically adapted and configured for an unsuitable section of artery or the like upstream of an aneurysm. These specific adaptations and configurations include, but are not limited to a first prosthesis having a highly flexible intermediate portion. As used herein, flexible or highly flexible refers to the capability of a stent to accommodate an artery having any angle. The typical prosthesis includes a stent having an intermediate portion, e.g., a section between the proximal and distal ends, that is formed of interconnected struts that can bend. In a preferred embodiment of the invention, the stent can bend without kinking.

A system according to the present invention is intended for repairing or bypassing an aneurysm, preferably an aortic aneurysm. The system may also be used to direct fluid flow from one portion of a fluid pathway to another. The system may also be used for repairing or bypassing aneurysms having an upstream portion unsuitable for anchoring or using a typical prosthesis.

The typical system according to the present invention may include multiple system components, e.g., more than one prosthesis, with the first prosthesis typically positioned upstream of an aneurysm. In preferred embodiments of the invention, the first prosthesis includes one or more structures that anchor system components in their proper position. The first prosthesis also preferably includes gasket material configured and adapted to facilitate delivery of other system components, to receive and/or position other system components, and/or to seal the system. In some, embodiments of the invention, e.g., those in which the system is positioned upstream of a cross artery, the first prosthesis also provides at least one fluid flow path.

For example, a system may include a first prosthesis configured to be positioned in an artery upstream of an aneurysm, and a second prosthesis that matingly engages the first prosthesis. The second prosthesis provides a fluid flow path that bypasses the aneurysm. As will be evident from the description below, the system may include a variety of other components all adapted to communicate with another component in the system, with a particular assembly of components designed to establish one or more fluid flow paths that bypass a pre-determined location, e.g., a location that includes an aneurysm and/or an arterial junction.

In some exemplary embodiments of the invention, the gasket material on the first prosthesis further includes one or more structures configured to assist in delivering one or more other components of the system into position.

The apparatus, systems and kits of the present invention may be used in the treatment of aortic aneurysms, preferably an abdominal aortic aneurysm, among other uses noted below.

The present invention is directed to a system for repairing, and/or bypassing an aneurysm comprising a first prosthesis comprising a gasket material engaging a stent, the gasket material and stent providing a seal for the system; the first prosthesis further comprising at least one manifold configured to receive at least one second prosthesis. In exemplary embodiments of the invention, the second prosthesis is configured for establishing a fluid flow path through the system and/or aneurysm. In most preferred embodiments of the invention, the manifold is configured to engage two second prostheses.

The present invention is also directed to a system that may further comprise a first prosthesis having at least one second manifold, the second manifold being configured to receive at least one third prosthesis. In exemplary embodiments of the invention, the third prosthesis is configured for establishing a fluid flow path into an artery upstream of the aneurysm. In most preferred embodiments of the invention, the second manifold is configured to engage two third prostheses. In the most preferred embodiments of the invention, the third prosthesis is configured for establishing a fluid flow channel from a proximal portion of the first prosthesis into a cross artery.

In some embodiments of the invention, the first manifold is configured to receive two second prosthesis, each of the second prostheses preferably having a distal end positioned in an artery downstream of the aneurysm (e.g., an iliac artery). In some embodiments of the invention, the second manifold is configured to receive two third prostheses, each of the third prostheses having a distal end positioned in an artery upstream of the aneurysm (e.g., a renal artery).

The present invention is also directed to a system for repairing an aneurysm, said system being variously configured and/or assembled using components described in more detail below. Typical systems according to this aspect of the invention may include one or more first prostheses or a sealing component, one or more second prostheses or a fluid flow component, and, optionally, one or more component receptacles, assemblies, or connectors for matingly engaging one component with another. Preferred embodiments of a system of the present invention include a sealing component matingly engaged to two fluid flow path components.

Any of the prostheses or stents described above may form a component or portion of a system or kit for bypassing an aneurysm.

Any of the prostheses, stents, systems, or kits described above may be used in a method for treating an aneurysm. In preferred embodiments of the invention, the prostheses, stents, systems, or kits are used to treat an aortic aneurysm, even more preferably, an abdominal aortic aneurysm.

The prostheses and stents can be used in a method which comprises delivering and deploying a first prosthesis upstream of an aneurysm, the first prosthesis being adapted to receive at least one second prosthesis and positioning a proximal end of at least one second prosthesis in a distal portion of the first prosthesis. In some embodiments of the invention, the method may include delivering and positioning a first manifold in a distal portion of the first prosthesis. In some embodiments of the invention, the method may include matingly engaging a proximal end of the second prosthesis with the first manifold. In some embodiments of the invention, the method may further include positioning a distal end of the second prosthesis in an artery downstream of the aneurysm.

In some embodiments of the invention, the method may also include delivering and positioning a second manifold in a proximal portion of the first prosthesis. In some embodiments of the invention, the method may include matingly engaging a proximal end of a third prosthesis with the second manifold. In some embodiments of the invention, the method may further include positioning a distal end of the third prosthesis in an artery upstream of the aneurysm.

In preferred embodiments of the invention, the method includes anchoring the system using the second prosthesis in its expanded configuration. The method may further include anchoring the most upstream portion of the system using the first portion of the stent, matrix, or first prosthesis.

Exemplary prostheses of the present invention may be configured to repair an abdominal aortic aneurysm. In these embodiments of the invention, the first prosthesis may be positioned in an infra-renal or supra-renal portion of the abdominal aorta, the second prosthesis may extend into one of the iliac arteries, and the third prosthesis may extend into one of the renal arteries.

The present invention is also directed to a kit that includes one or more of the following: a sterile or sterilisable enclosure; a first prosthesis; a first prosthesis in an individual sterile enclosure; a second prosthesis; a second prosthesis in an individual sterile enclosure; a third prosthesis; a third prosthesis in an individual sterile enclosure; at least one suture; at least one staple; a collar or catheter tip assembly configured to engage and deliver a first prosthesis, a second prosthesis, and/or a third prosthesis; and at least one marker configured for placement on a first prosthesis, a second prosthesis, a third prosthesis, and/or portions thereof.

The present invention also includes a kit comprising a prosthesis according to the invention, preferably in a sterile or sterilisable enclosure.

A system or kit of the present invention may include one or more modular components. As used herein, a modular component is configured, or adapted to engage, or includes one or more structures that are intended to communicate with or engage a complementary structure on another modular component. The present invention also includes a kit that includes one or more of the following: a sterile or sterilisable enclosure; a first prosthesis; a first prosthesis in an individual sterile enclosure; a second prosthesis; a second prosthesis in an individual sterile enclosure; a third prosthesis; a third prosthesis in an individual sterile enclosure; at least one suture; at least one staple; a collar or catheter tip assembly configured to engage and deliver a first prosthesis, a second prosthesis, and/or a third prosthesis; and at least one marker configured for placement on a first prosthesis, a second prosthesis, a third prosthesis, and/or portions thereof.

Embodiments of the invention may further include one or more second and/or third prostheses configured to matingly engage a first prosthesis, the second and/or third bypass prosthesis comprising a graft material engaging a stent, the stent comprising a hollow matrix comprising a series of interconnected struts, the matrix being moveable from a first closed position to a second open position; the stent having at least one attachment structure or connector for matingly engaging at least one second complementary structure on the first prosthesis. In some embodiments of the invention, the prosthesis further comprises at least one marker. In preferred embodiments of the invention, the marker or markers are positioned on or formed as part of the stent.

Other embodiments of the invention will be evident from the description provided below.

### DEFINITIONS

As used herein, aortic aneurysm refers to any failure of a conduit, such as an aortic wall, typically characterized by an undesirable dilation of a portion of the artery, vessel malformation, or an occlusion. The system and structures of the present invention may be used to treat, repair, replace, or bypass any blood vessel (e.g., artery, vein, capillary); any fluid carrying vessel (e.g., lymphatic vessels); any organ or portion thereof that includes a blood or fluid vessel; or any junction between blood vessels, between fluid vessels, and between organs and blood vessels. An exemplary use of a system of the present invention is to repair an aortic aneurysm, and the use of such term is not intended to limit the use of the structures or systems of the present invention to repair or replace other conduit failures. The prosthesis of the present invention may also be utilized in the thoracic aorta, and can be used to repair thoracic aneurysms or thoracic dissecting aneurysms. Accordingly, use of the term "aortic aneurysm" is intended to relate to and include other aneurysms, including but not limited to both abdominal aortic aneurysms and thoracic aneurysms.

In preferred embodiments of the invention, the system and structures are used to treat, repair, replace, or bypass an abdominal aortic aneurysm.

As used herein fluid pathway refers to any *in vivo* structure through which a biological fluid passes. A preferred fluid pathway is an artery. Fluid pathways include, but are not limited to channels formed by an artery, a vein, a capillary, lymph nodes and channels, and arteries, veins, and capillaries within an organ or organelle.

As used herein fluid or biological fluid refers to any fluid produced by an animal, including a human. Exemplary biological fluids include but are not limited to blood, oxygenated blood, de-oxygenated blood, gastric fluids, amniotic fluid, spinal fluid, and lymph. The preferred fluid is blood or oxygenated blood. As used herein, conduit typically refers to any structure used to convey a biological fluid. The conduit may be formed of natural or synthetic materials, or combinations thereof. Exemplary conduits include but are not limited to an artery, a vein, a capillary, lymph nodes and channels, and arteries, veins, capillaries within an organ or organelle, and a prosthesis or system according to the invention.

As used herein, "biofusion" refers to the ability of cells, proteins, fibrin, and other biological molecules to incorporate into the pore structure of a material, such as a foam or gasket material, or a graft material. It is believed that this feature promotes a long term stable biological interface that cannot be separated about six weeks after implantation.

The biofusion effect has many advantages. It has the potential to obviate late endo-leakage by preventing areas of non-organized clot from being displaced or recanalised. It is also believed that biofusion creates a connective tissue collar around the prosthesis that may prevent the aortic neck from dilating over time. Restricting neck dilation avoids leakage pathways and implant migration that can be caused by an insufficient fit with the aorta.

As used herein, adapted for communication, communicating, or similar terms refer to any means, structures, or methods for establishing operational association between two elements of the system. Similarly, engaging, adapted to engage, or similar terms refer to means, structures, or methods for contacting a first component, structure, or portion thereof with a second component, structure, or portion thereof. Exemplary structures are shown in the figures. Typically, all of these terms and phrases refer to at least one structure in or on a first component configured to engage a complementary structure in or on a second component, and the use of these inter-engaging features to link a first prosthesis or component with a second prosthesis or component. The engagement or communication may be matingly (e.g., permanent) and/or releasably (e.g., temporary). In preferred embodiments of the invention, communication or engagement may be fluid tight, substantially fluid tight, or fluid tight to an extent so as to not substantially compromise the intended function of the structure.

For example, a connector may be adapted to receive or connect to a complementary connector on another prosthesis. As used herein, connector refers to any structure used to form a joint or to join itself to another component or portion thereof. These connectors or connections establish a fluid flow path through various elements of the apparatus, assembly, or system. In a preferred embodiment of the present invention, the system is intended to establish at least one fluid flow path through a vessel, conduit, organ, or portions thereof. Typical connections include but are not limited to mating connections, such as Luer-type, screw-type, friction-type, or connectors that are bonded together.

As used herein, distal is used in accordance with its ordinary dictionary definition, e.g., referring to a position farthest from the beginning; in human anatomy, this term is commonly equivalent to caudal or inferior. Proximal is used in accordance with its ordinary dictionary definition, e.g., referring to a position nearest the beginning; in human anatomy, this term is commonly equivalent to cranial or superior. The terms distal and proximal are intended to convey opposite ends or portions of a device, channel, element, or structure. In relation to a fluid flow path, distal will typically refer to a downstream location in the fluid flow path, and proximal will typically refer to an upstream location, unless otherwise specifically noted. Anatomically, distal generally refers to "away from the heart" and proximal generally refers to "toward the heart".

A system for treating an aortic aneurysm according to the present invention typically includes a first prosthesis defining a lumen therethrough, a manifold positioned in the first prosthesis across the lumen, and at least one second prosthesis. In preferred embodiments of the invention, the components of the system are delivered percutaneously and/or intraluminally to the site of the aneurysm using a catheter or the like. One skilled in the art will therefore recognize that it is beneficial to deliver the components of the system in an unexpanded or crimped first position, and to deploy the component in its functional location by deploying the component into an expanded or second position. A typical second prosthesis forms a fluid flow channel that bypasses the aneurysm. The system may also include at least one third prosthesis, typically forming a fluid flow path into a cross artery upstream of the aneurysm.

Embodiments of the invention will now be described by way of example with reference to the accompanying drawings, in which:
Figure 1 is a view of a fully deployed aortic repair system made in accordance with the present invention.
Figure 2 shows the fully deployed alternative system positioned upstream of a junction with another artery.
Figure 2A is a sectional view, taken along section line 2A-2A, of the first prosthesis illustrated in Figure 2.
Figure 3 is a side elevation of a first, second, and/or third prosthesis according to the invention, each having a stent covered by a graft material.
Figure 4 is a side elevation of an embodiment of a stent of the present invention.
Figure 5 is a side elevation of a graft material of the present invention.
Figure 6 is an end view of the graft material illustrating the graft material in its unexpanded or crimped configuration, and in its fully expanded configuration.
Figure 7 is a partial exploded perspective view of the distal end of a prosthesis of the present invention, illustrating an exemplary anchoring and delivery system according to the invention.
Figure 8 is a view of a fully deployed alternate embodiment of an aortic repair system made in accordance with the present invention.
Figure 9 is a view of a fully deployed alternate embodiment of an aortic repair system made in accordance with the present invention.

### SYSTEM

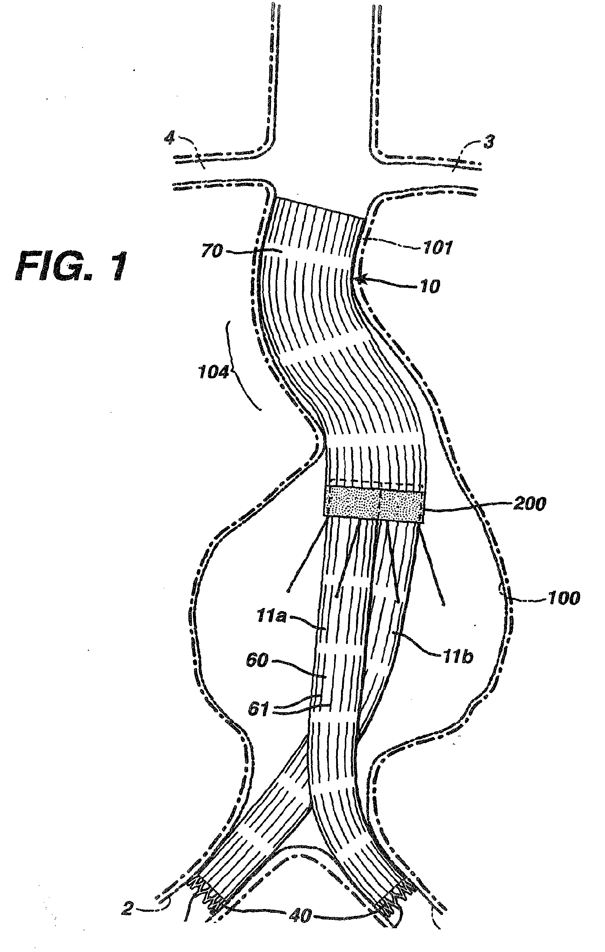

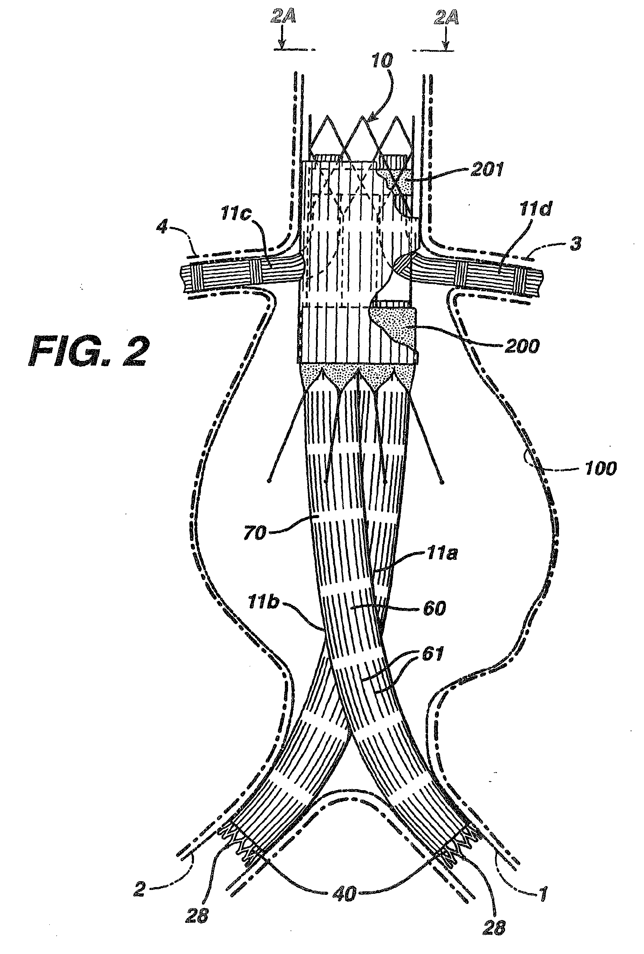

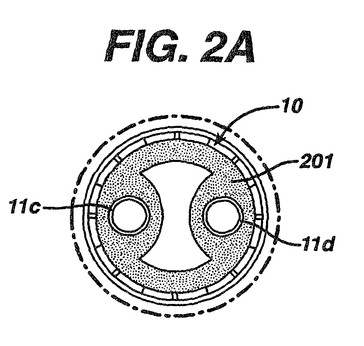

A system according to the present invention may include one or more prostheses. Exemplary systems are shown in Figures 1, and 2A. The system includes a first prosthesis 10 and at least one second prosthesis, preferably two second prostheses 11a and 11b, which, in combination, bypass an aneurysm 100. In preferred embodiments of the invention, a proximal portion of the system may be positioned in a section of an artery upstream of the aneurysm 100, and a distal portion of the system may be positioned in a down stream section of the artery or a different artery. Some embodiments of the system may also include at least one third prosthesis (Figure 2), preferably two third prostheses 11c and 11d, which may be configured to provide a fluid flow channel into an artery or the like upstream of the aneurysm, e.g., a renal artery 3 or 4.

As shown most clearly in Figure 1, the system of the present invention may be used when the upstream section of the artery is unsuitable for anchoring a portion of the system. As noted above, these circumstances exist when the length of the upstream section is diseased, too short, includes a junction with a second artery 3 or 4, and/or includes angulated sections 104 of artery. Under these and other circumstances, it may be desirable to provide a system, first prosthesis, or stent having a proximal portion that extends into an upstream portion of the artery. This proximal portion anchors the system or prosthesis in a section of the artery that is suitable for engaging and anchoring the system or prosthesis.

As shown in Figure 2, it may also be beneficial to provide a system having one or more third prostheses for channelling fluid flow into a cross or second artery 3 or 4. Under these and other circumstances, it may be desirable to provide a system, first prosthesis, or stent positioned across the junction between two or more arteries. This proximal portion anchors the system or prosthesis in a section of the artery that is suitable for engaging and anchoring the system or prosthesis, and may be further adapted to receive various other prostheses for bypassing the aneurysm and/or establishing fluid communication with one or more arteries upstream of the aneurysm.

A prosthesis of the present invention includes a support, stent, or lattice of interconnected struts defining an interior space having an open proximal end and an open distal end. The lattice also defines an interior surface and an exterior surface. The interior and/or exterior surfaces of the lattice, or a portion of the lattice, may be covered by or support at least covering material, such as a foam or graft material.

As noted in more detail below in relation to specific system components, some prostheses of the present invention may be configured to seal and/or anchor the system in place, and/or to receive and position other prostheses. Typically these prostheses do not themselves define a fluid flow path. Other prostheses may be configured to define at least one fluid flow path. Typically, these prostheses define a channel or the like through which fluid, such as blood, flows. This channel or fluid flow path typically begins upstream of, or in an upstream portion of, a component of the system. In some embodiments of the invention, the fluid flow path bypasses the aneurysm.

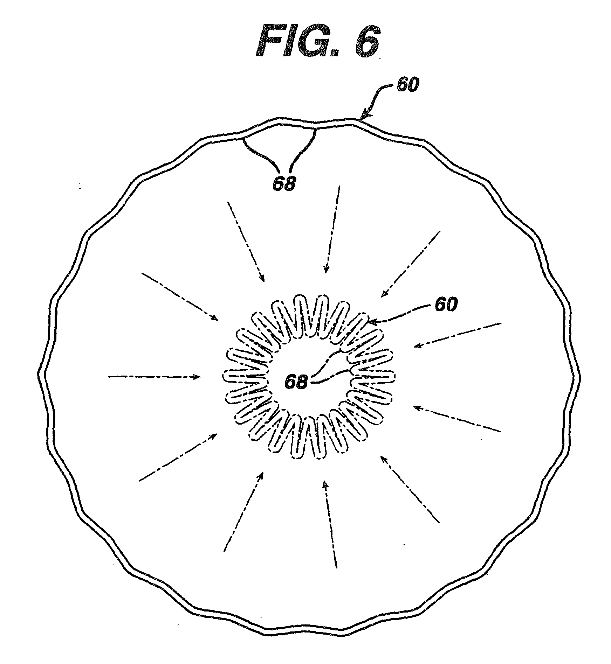

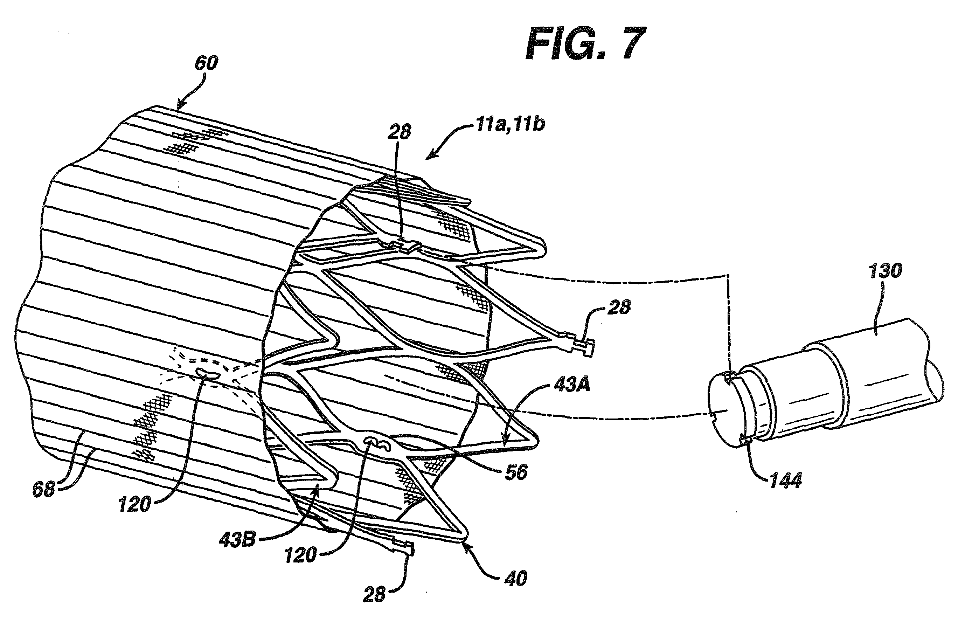

In preferred embodiments of the invention, a prosthesis is moveable between an expanded or inflated position and an unexpanded or deflated position, and any position therebetween. An exemplary embodiment of this feature of the invention is shown in Figure 6. In some embodiments of the invention, it may be desirable to provide a prosthesis that moves only from fully collapsed to fully expanded. In other embodiments of the invention, it may be desirable to expand the prosthesis, then collapse or partially collapse the prosthesis. Such capability is beneficial to the surgeon to properly position or re-position the prosthesis. In accordance with the invention, the prosthesis may be self-expanding; or may be expandable using an inflatable device, such as a balloon or the like. Even further in accordance with the present invention, there is provided a delivery apparatus for a self-expanding prosthesis. As shown in Figure 7, the delivery apparatus may include an outer sheath, comprising an elongated tubular member having distal and proximal ends, and an inner shaft located coaxially within the outer sheath, the shaft having a distal end and a proximal end. The distal end of the shaft may further include at least two grooves disposed thereon. The flanges of the first prosthesis may be configured to releasably engage the grooves of a portion of the delivery device.

Exemplary embodiments of a system for treating an abdominal aortic aneurysm according to the present invention are shown in Figures 1 and 2. For the purpose of this embodiment, the system is deployed in the infrarenal neck of the abdominal aorta, upstream of where the artery splits into iliac arteries. Figure 1 shows first prosthesis 10 positioned in the infrarenal neck; two second prostheses, 1 1a and 11b, the proximal ends of which matingly engage a manifold 200 positioned in a distal portion of first prosthesis 10, and the distal ends of which extend into an iliac artery 1 or 2. As illustrated, the body of the prosthesis forms a conduit or fluid flow path that passes through the location of the aneurysm 100. In preferred embodiments of the invention, the components of the system define a fluid flow path that bypasses the section of the artery where the aneurysm is located.

Alternately, Figure 2 shows first prosthesis 10 positioned in the supra- renal portion of abdominal aorta 302. Two prostheses, 1 1a and 11b, the proximal ends of which are in fluid communication with the first prosthesis 10, and the distal ends of which extend into an iliac artery 1 or 2, bypass aneurysm 100. Two other prostheses, 11c and 11d, the proximal end of which matingly engage a proximal portion of first prosthesis 10, and the distal ends of which extend into a renal artery 3 or 4. As illustrated, the body of the prosthesis 11a and 11b forms a conduit or fluid flow path that passes through the location of the aneurysm 100; and the body of the prosthesis 11c and 11d forms a conduit or fluid flow path that passes into an artery upstream of the aneurysm. In preferred embodiments of the invention, the components of the system define a fluid flow path that bypasses the section of the artery where the aneurysm is located.

These and other features of the prosthetic devices and systems of the present invention will be described in more detail below.

### FIRST PROSTHESIS

First prosthesis is typically deployed in an arterial passageway upstream of an aneurysm, and functions to open and/or expand the artery, to properly position and anchor the various components of the system, and to establish or form the beginning of one or more fluid flow paths or channels. The first prosthesis typically includes a support matrix or stent that supports a graft material. The first prosthesis may further include one or more structures for engaging and fixing at least one second prosthesis in place.

The first prosthesis may further include a manifold or a compressible gasket located within the interior of the prosthesis, typically across the fluid flow path, over all or a part of the distal end of the prosthesis. The manifold may be formed as an integral part of the graft material, or may be separate. In preferred embodiments of the invention, the manifold includes one or more holes, apertures, slits, sleeves, guides or the like for positioning a guidewire, for positioning a system component, such as a second prosthesis, and/or for engaging, preferably matingly engaging, one or more system components, such as a second or third prosthesis. The manifold is preferably substantially impervious to blood when in a compressed state. These and other features of the first prosthesis will be described in more detail bellow.

First prosthesis 10 is typically deployed in an artery, upstream of an aneurysm. For example, the first prosthesis may be deployed within the infrarenal neck, between an abdominal aortic aneurysm and the renal arteries of a patient to assist in repairing an abdominal aortic aneurysm (see Figure 1). As shown in Figures 2, and 2A first prosthesis 10 may also be positioned upstream of an aneurysm and upstream of a branch or cross artery. In these embodiments of the invention, the first prosthesis will also include one or more elements for establishing fluid flow into the branch arteries, described in more detail below.

The first prosthesis typically includes a support matrix or stent that supports a graft material. One end of the first prosthesis is typically adapted to engage one or more portions of a second prosthesis. In preferred embodiments of the invention, the proximal end of second prosthesis is adapted to matingly engage a distal portion of first prosthesis.

Figures 1, 2 and 2A show exemplary embodiments of the first 10 prosthesis of the present invention. First prosthesis 10 includes a substantially cylindrical self-expanding lattice, support, or stent, typically made from a plurality of interconnected struts. The lattice defines an interior space having two open ends, a proximal end and a distal end. The interior and/or exterior surfaces of lattice may be covered by or support at least one graft material. These and other features of the first prosthesis will be described in more detail below.

One or more markers may be optionally disposed in or on the stent between the proximal end and the distal end. One or more markers may be optionally disposed in or on the stent between the proximal end and the distal end. Preferably, two or more markers are sized and/or positioned to identify a location on the prosthesis, or to identify the position of the prosthesis, or a portion thereof, in relation to an anatomical feature or another system component. In exemplary embodiments of the invention, fluoroscopically identifiable sutures or staples may be used. These sutures or staples may also attach the graft material to the stent.

In some exemplary embodiments of the invention, the graft material may cover the lattice from the distal end to the proximal end. In some exemplary embodiments of the invention, the graft material may also cover, or include a cover, over the proximal end of first prosthesis 10. The cover may also include one or more holes or openings. These openings may be variously configured, primarily to conform to its use. For example, the openings may be a hole, aperture, slit, point, or weakened spot in the cover. Further, these openings may exist prior to deployment of the prosthesis, or may be formed in the prosthesis as part of a deployment procedure. The various functions of the openings are described in more detail below.

In some exemplary embodiments of the present invention, the first prosthesis 10, further includes a first manifold 200 and/or a second manifold 201. This feature can be better understood by referring to Figures 1, 2 and 2A. The manifold helps position one or more second prostheses, and to seal or impede any blood trying to flow around second prostheses 11a and 11b after they have been deployed (as shown in Figure 1).

A manifold may be made from any number of materials known to those of ordinary skill in the art, including but not limited to open cell foam materials such as polyurethane, polyethylene, polytetrafluoroethylene, and other various polymer materials, preferably woven or knitted, that provide a flexible structure, such as a polyester (such as that sold under the trade mark Dacron). Highly compressible foams are particularly preferred, preferably to keep the crimped profile low for better delivery. A manifold may be attached to the stent by any number of connectors, including a plurality of conventional sutures of polyvinylidene fluoride, polypropylene, polyester, or any other suitable material and attached thereto. Other methods of attaching the manifold to expandable member include adhesives, ultrasonic welding, mechanical interference fit and staples.

In other embodiments of the invention, the sealing function of the manifold may by embodied in a gasket or ring material surrounding the outside of one end of the second prosthesis. In these embodiments of the invention, when the second prosthesis is expanded, the gasket or ring engages the inside wall of the first prosthesis in a fluid tight seal.

One skilled in the art will also recognize that some of the "straight" embodiments described above may be used in pathological conditions that involve or need an angled blood or fluid flow path. For example, a straight prosthesis may be used when only a small angle is involved. Any of the straight embodiments described above may be deformed to achieve an angled fluid flow path if the amount of deformation does not adversely affect the function of the prosthesis or the well being of the patient.

In accordance with the present invention, the upstream portion, component, or prosthesis of the system may be variously configured to achieve a flexible structure suitable for accommodating one or more highly angled sections of an artery. In preferred embodiments of the invention, the flexibility is achieved without creating kinks in the structure. The upstream portion, component, or prosthesis of the system may include open or unattached diamonds or struts, resilient struts, or the like. In preferred embodiments of the invention, the stent or matrix configuration is flexible both longitudinally and radially. As used herein, longitudinal flexibility refers to the ability for a stent or matrix to shorten or elongate as needed.

Conversely, one skilled in the art will recognize that a pathological or biological condition having a fluid flow path from a slight deflection to a wide angle (e.g., from about forth-five degrees to about ninety degrees) may warrant the use of a prosthesis having a structural configuration or element that allows the prosthesis to achieve the angled configuration. In these situations, it is believed that the following are exemplary embodiments of the invention that would provide beneficial results in achieving a fluid flow path through a tortuous channel.

A prosthesis having an angled conformation may be achieved by interposing one or more pivots, joints, axes, junctions, hinges, narrows, hubs, or the like, in the matrix or lattice that forms the stent. In preferred embodiments of the invention, an intermediate portion of the stent is configured into a series of interconnected struts having greater flexibility. In accordance with the present invention, greater flexibility may be achieved by changing the material used to form the matrix, and/or preferably, by changing the configuration of the diamonds.

In other embodiments of the invention, the struts of the stent may be configured to achieve a flexible structure, e.g., open diamonds or unconnected diamonds, to name two exemplary configurations, may provide the stent with longitudinal and/or radial flexibility.

An alternate exemplary embodiment of the present invention uses a first prosthesis 10 as described for Figure 1, and positions it across an arterial junction, as shown in Figure 2. As is readily evident to one skilled in the art, a system that includes a first prosthesis 10 upstream of both an aneurysm and cross arteries will preferably include a number of second and third prostheses for establishing alternate fluid flow paths. In the exemplary embodiment shown in Figure 2, the system includes two second prostheses, 11a and 11b, and two third prostheses, 11c and 11d.

As shown in Figures 1 and 2, one or more manifolds may be configured to receive one or more additional system components by including one or more slits, holes, passages, cavities, or the like. Preferably, any structure configured to receive another system component will be deformable or resilient to sealingly engage a portion of the system component.

### SECOND PROSTHESIS

The second prosthesis is a bypass conduit or the like that is typically deployed in an arterial passageway upstream of an aneurysm, and extends from a position upstream of an aneurysm, e.g., a healthy portion of the artery, through the arterial segment having the aneurysm, and into a position downstream of the aneurysm. The second prosthesis functions to bypass the portion of the conduit containing the aneurysm, and to properly position and/or anchor the proximal end of the system in an artery. The second prosthesis may also include one or more structures for positioning and anchoring the second prosthesis in the artery or in the first prosthesis. In a preferred embodiment of the invention, the second prosthesis is adapted to engage the first prosthesis.

The second prosthesis typically includes a support matrix or stent that supports a graft material. One end of the second prosthesis is typically adapted to engage one or more portions of the first prosthesis. In preferred embodiments of the invention, the proximal end of second prosthesis is adapted to matingly engage a proximal portion of the first prosthesis. The second prosthesis may optionally include at least one attachment structure on its distal end for engaging and securing the prosthesis in a portion of an artery downstream of the aneurysm.

Figures 1 to 5 show exemplary second or bypass prostheses 11a,b of the present invention. Second prosthesis 11a,b includes a substantially cylindrical self-expanding lattice, support, or stent 40, typically made from a plurality of interconnected struts 44. Lattice 40 defines an interior space having two open ends, a proximal end 41 and a distal end 42. The interior and/or exterior surfaces of lattice 40 may be covered by or support at least one graft material 60. These and other features of the second prosthesis will be described in more detail below.

### THIRD PROSTHESIS

A third prosthesis is a second prosthesis that does not pass through the aneurysm. The third prosthesis is a bypass conduit or the like that is typically deployed in an arterial passageway upstream of an aneurysm, and extends from a healthy portion of a first artery into another healthy portion of the first artery or into a second or branch artery. The third prosthesis functions to establish a fluid flow path or channel from an upstream portion of the system into an artery upstream of the aneurysm, and to properly position and/or anchor a proximal end of the system in an artery. The third prosthesis may also include one or more structures for positioning and anchoring the third prosthesis in the artery or in the first prosthesis. In a preferred embodiment of the invention, the third prosthesis is adapted to engage the first prosthesis.

Figure 2 shows exemplary third prostheses 11c and 11d of the present invention. Any third prosthesis may be configured as described above for any second prosthesis.

### STENT

Any of the stents of the present invention form a support or lattice structure suitable for supporting a graft material. In preferred embodiments of the invention, the stent defines a channel or lumen through which a fluid, such as blood, may flow. A typical stent comprises an expandable lattice or network of interconnected struts. In preferred embodiments of the invention, the lattice is fabricated, e.g., laser cut, from an integral tube of material.

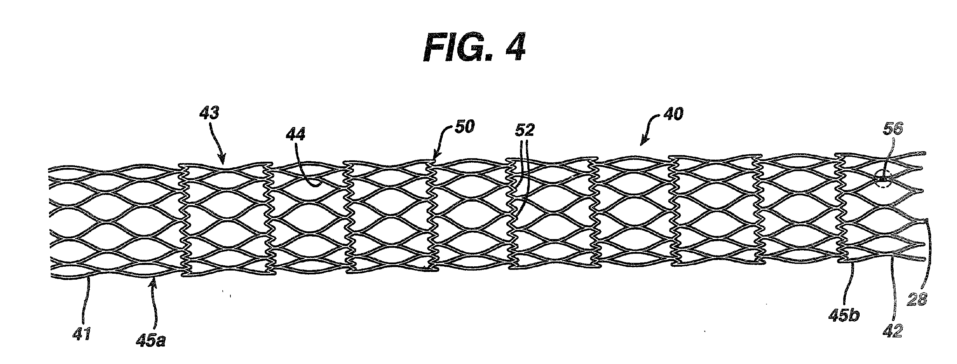

In accordance with the present invention, the stent may be variously configured. For example, the stent may be configured with struts or the like that form repeating geometric shapes. One skilled in the art will readily recognize that a stent may be configured or adapted to include certain features and/or to perform a certain function(s), and that alternative designs may be used to promote that feature or function. In the exemplary embodiment of the invention shown in Figure 4, the struts 44 of stent 40 are configured into a diamond shape.

In the embodiment of the invention shown in Figure 4, the matrix or struts of stent 40 may be configured into at least two hoops 43, each hoop 43 comprising a number of struts 44 having a diamond shape. A second and/or third prosthesis, such as second prosthesis 11a,b, may further include a zigzag shaped ring 50 for connecting adjacent hoops to one another. The sinusoidal rings may be formed from a number of alternating struts 52.

The diamond pattern for the hoops provide the hoops with radial and longitudinal stiffness. The longitudinal strength provides for better mechanical fixation of stent 40 to a graft material (described below). The radial strength provides the proximal hoop 45a with better attachment and sealing to the graft material, and provides the distal hoop 46b with better fixation and sealing to the arterial wall. Further, the distal hoop may be flared, and may be exposed after the graft material has been attached to the stent.

In one exemplary embodiment, the proximal and distal hoops have greater radial and longitudinal strength than the hoops therebetween. This creates a stent graft having stiff ends for anchoring, but a more flexible body for navigation through the vasculature. The stiffer ends may be accomplished by changing the dimensions of the struts for the end hoops, or by varying the heat treatment of the end hoops during manufacture. The rings allow the stent to bend more easily, and generally provide for more flexibility when the stent is being delivered through a tortuous vessel. When a non-compliant graft is attached to stent 40, the strength of the diamond hoops scaffolds any graft folding into the blood flow lumen, while maintaining a tight kink radius.

A flexible stent structure, wherein the flexibility is derived from the bridge and/or strut configuration itself, may provide sufficient flexibility and/or articulation to accommodate extreme angulations in an artery's shape. These various flexible stent structures are also included in the meaning of jointed stent.

Any of the stents of the present invention may be formed of any material suitable for functioning *in vivo* as a support for graft material. A stent of the present invention may be formed of a wide variety of materials, all of which are well known to those skilled in the art. In some embodiments of the invention, the stent is formed from a metal or metal alloy. In preferred embodiments of the invention, the stent is formed from superelastic Nickel Titanium alloys (Nitinol). Descriptions of medical devices which use such alloys can be found in US-4665906 and EP-A-928606. A stent according to the invention is preferably laser cut from a tubular piece of nitinol and thereafter treated so as to exhibit superelastic properties at body temperature. In preferred embodiments of the invention, the stent material is expandable or collapsible, i.e., moveable from a first closed position to a second open position, or vice versa.

The distal end of the stent is preferably configured to engage a complementary structure on a delivery device, such as a catheter or a portion thereof. For example, the distal end of the stent may include one or more keys that engage, preferably releasably engage, a corresponding latch on the catheter. An exemplary configuration is shown in Figure 7. It is intended that the invention should not be limited by the precise structures used to engage the stent to the delivery device.

In the exemplary embodiments of the invention shown in the Figures, the stent may include one or more anchors flanges 28 configured to engage a corresponding structure on a delivery device 130 (illustrated most clearly in Figure 7). In accordance with the present invention, the delivery apparatus 130 may include a collar having one or more grooves or the like adapted to releasably engage one or more complementary structures on a stent or prosthesis of the present invention. For example, the delivery apparatus shown in Figure 7 includes three grooves 144 to configure the delivery device to releasably engage the second prosthesis 1 1a, 11b in Figure 1 (having three anchors 28), and the third prosthesis 11c, 11d in Figure 2 (having three anchors 28). Such an anchor/delivery device configuration is particularly suited to partially deploying a prosthesis of the present invention, and to position or re-position the prosthesis.

### GRAFT MATERIAL

An inner and/or outer surface of a stent of the present invention is preferably covered by a graft material. As noted above, graft materials or fabrics are well known to those skilled in the art. Graft material 60 may be made from any number of materials known to those skilled in the art, including woven polyester, polyester (such as that sold under the trade mark Dacron), polytetrafluoroethylene (such as that sold under the trade mark Teflon), polyurethane, porous polyurethane, silicone, polyethylene terephthalate, and expanded polytetrafluoroethylene (ePTFE) and blends of various materials.

In some exemplary embodiments of the invention, it may be desirable to incorporate a biodegradable, or degradable material, such as albumin, collagen, or any type of collagen. A graft material that is biodegradable would erode or dissolve over time; however, it is believed that a layer of endothelium may grow as the graft material erodes. It is further believed that these new layers of endothelium may provide a new, fluid impervious lining within the aneurysm.

It is preferred that all of the foregoing materials be porous to allow for an intimal layer to form a biofusion structure or matrix.

The graft material may be variously configured, preferably to achieve predetermined mechanical properties. For example, the graft material may incorporate a single or multiple weaving and/or pleating patterns, or may be pleated or unpleated. For example, the graft may be configured into a plain weave, a satin weave, include continuous longitudinal pleats, interrupted pleats, annular pleats, radially oriented pleats, or combinations thereof. Alternately, the graft material may be knitted or braided. In the embodiments of the invention in which the graft material is pleated, the pleats may be continuous or discontinuous. Also, the pleats may be oriented longitudinally, circumferentially, or combinations thereof.

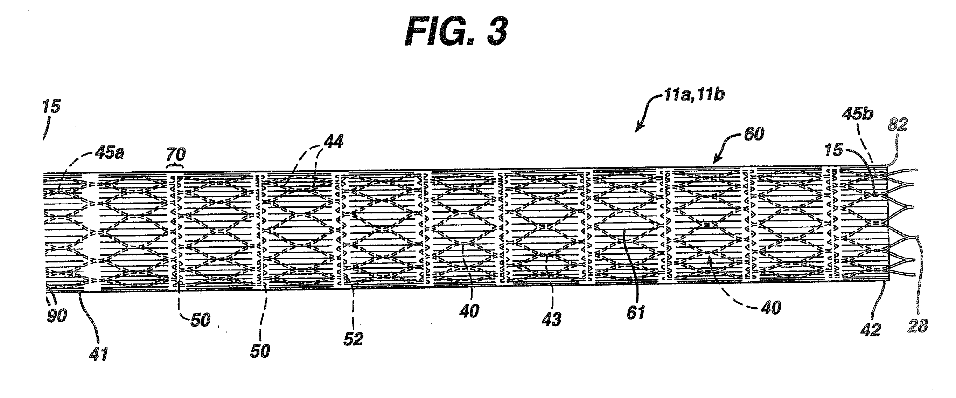

As shown in Figure 3, graft material 60 may include a plurality of longitudinal pleats 61 extending along its surface, generally parallel to the longitudinal axis of the prosthesis. As shown in Figure 6, the pleats allow the prosthesis to collapse around its centre, much as it would be when it is delivered into a patient. As illustrated, the pleats come together as a series of radially oriented regular folds 68 that pack together efficiently. This provides a relatively low profile delivery system, and provides for a controlled arid consistent deployment therefrom. It is believed that this configuration minimizes wrinkling and other geometric irregularities. Upon subsequent expansion, the prosthesis assumes its natural cylindrical cross-sectional shape, and the pleats or folds uniformly and symmetrically open.

In addition, pleats 61 help facilitate stent graft manufacture, in that they indicate the direction parallel to the longitudinal axis, allowing stent to graft attachment along these lines, and thereby inhibiting accidental twisting of the graft relative to the stent after attachment. The force required to push the stent-graft out of the delivery system may also be reduced, in that only the pleated edges of the graft make frictional contact with the inner surface of the delivery system. One further advantage of the pleats is that blood tends to coagulate generally uniformly in the troughs of the pleats, discouraging asymmetric or large clot formation on the graft surface, thereby reducing embolus risk.

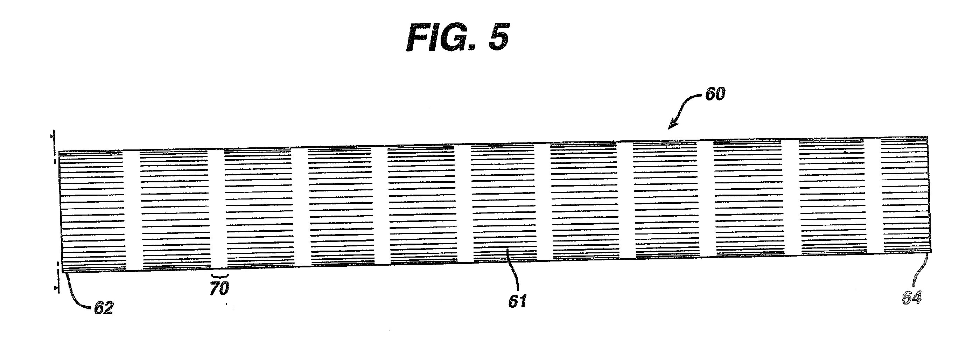

As shown in Figures 3 and 5, the graft material may also include one or more, and preferably a plurality of, radially oriented pleat interruptions 70. The pleat interruptions are typically substantially circular and are oriented perpendicular to longitudinal axis. Pleat interruptions 70 allow the graft and prosthesis to bend better at selective points. This design provides for a graft material that has good crimpability and improved kink resistance.

As noted above, the prostheses may be pleated longitudinally, axially, or combinations of both. Under typical conditions, these pleats will form a relatively consistent pattern, e.g., pleats all of a certain length. In the exemplary embodiments of the present invention for use in a highly angulated artery, it may be desirable to vary the pattern or patterns of pleats. For example, in the area of greatest angle, it may be desirable to provide an extension prosthesis having one or two (or more, as needed) pleat interruptions or axially pleated sections separated by a shorter longitudinally pleated section or sections. It is believed that increasing the number of axial pleats in the highly angulated section of the artery reduces stress on the prosthesis, and may promote a more fluid tight fit of the system.

The graft material as described above is preferably highly compressible, which also promotes a low crimped profile for better delivery characteristics.

In accordance with the present invention, the graft material may be impervious or substantially impervious to the flow of blood, or may be porous. A graft material is impervious if it prevents blood from passing through the graft material on contact with blood or after the graft material is saturated with blood. Choice of the flow characteristics of a graft material are well known to those skilled in the art, and are tied in part to the intended function of the prosthesis or portion of the prosthesis. For example, it may be desirable for the graft material that forms the cover of the first prosthesis to be impervious or substantially impervious to the flow of blood. Alternately, it may be desirable for a graft material to be porous or partially porous to promote biofusion.

A graft material may be attached to a stent or to another graft material by any number of structures or methods known to those skilled in the art, including adhesives, such as polyurethane glue; a plurality of conventional sutures of polyvinylidene fluoride, polypropylene, polyester, or any other suitable material; ultrasonic welding; mechanical interference fit; staples, rivets, or the like. In preferred embodiments of the invention, the connector is a suture or staple, even more preferably, having a knotted or nub end. Further, a connector may be formed from a radiopaque material or a fluorescent material, each of which allow the connector to be used as a marker.

In accordance with the present invention, it may be highly desirable to provide a graft material that limits or eliminates the amount of blood that passes between the graft and the arterial wall, to provide a catheter-delivered graft or prosthesis that extends through a longer portion of an artery, to improve the anchoring mechanisms between two prostheses, to improve the anchoring mechanism between the prosthesis and the arterial wall or an interluminal cavity within an artery, and to improve the fluid dynamic and performance characteristics if the implanted prosthesis.

In an alternate design, graft material may not be utilized on either end of the stent. For example, on any endolegs, prostheses, extension cuffs, stent gaskets or other covered stents, both ends thereof may be left uncovered. The body has the ability to cover the exposed portions of the stent with endothelial cells and thus these exposed portions become endothelialised or incorporated into the vessel wall. This may be an important factor in the long term stability of the system. Essentially, over long periods of time, the aneurysmal sac can and will shrink if it is totally excluded from blood flow. This shrinkage changes the morphology of the aortic region that has been treated with the bypass prosthesis. If all ends of the system are firmly anchored in the actual vessel, as is the case when the ends are covered with endothelium cells, the system will be better able to withstand these morphological changes.

### MARKER

As noted above, a stent and/or prosthesis of the present invention may include one or markers. One skilled in the art will recognize that one or more markers may be positioned on the stent, the graft material, or on the prosthesis. In preferred embodiments of the invention, the markers are used to identify the position of the stent or prosthesis in relation to a body part and/or in relation to another stent or prosthesis, and/or to identify the position of one part of the prosthesis relative to another part. In most preferred embodiments of the invention, the marker(s) is used to identify a position *in vivo*.

As shown in Figure 4, a stent, such as stent 40, preferably includes one or more radiopaque markers 15. Exemplary materials for forming markers include but are not limited to tantalum, platinum, iridium, and gold. As shown, markers 15 are coils of radiopaque metal, wrapped around the struts of the stent. Markers 15 are preferably made from 0.19 mm (0.0075 inch) diameter tantalum (Ta) wire wrapped tightly around the struts. The number, location, and size of the marker may vary, and the markers be used alone or in combination to identify the position of a particular portion of the prosthesis. For example, a first distal marker may be five mm long and a second distal marker may be two mm long. Also, two distal markers may be one hundred eighty degrees apart, and a proximal marker may be positioned equidistant from each of the distal markers. In this exemplary configuration, the proximal marker then aids proper rotational positioning of the device.

### CONNECTORS

Some embodiments of a prosthesis according to the present invention may include one or more connectors. In some embodiments of the invention, the connectors are used to engage or connect one prosthesis or component to another. In some embodiments of the invention, the connectors may be used to attach the graft material to a stent or lattice.

As noted above, one skilled in the art will recognize that a variety of materials and methodologies may be used to connect one prosthesis to another, or to attach the graft material to a stent. Exemplary connectors include but are not limited to sutures and staples. Further, a connector such as a suture, staple, rivet, or the like may be formed from a radiopaque material or a fluorescent material, each of which allow the suture, etc., to be used as markers.

In accordance with the present invention, it may be desirable to incorporate in a prosthesis a connector adapted for use with a lattice-like stent. A first connector 54, an exemplary embodiment of which is shown in Figure 4, is configured for use at an end portion of a stent, preferably at an end portion of a strut 44. A second connector 56, an exemplary embodiment of which is shown in Figure 7, is configured for use at an internal portion of a stent, preferably at the junction between two struts 44.

A connector configured for receiving a rivet, staple, suture, or the like, may include two apertures, each aperture configured to receive a leg of the rivet, staple, suture, or the like. In this embodiment of the invention, the end of each leg is preferably formed into a knot, nub, or spherical end that is of larger diameter than the diameter of the aperture. Preferably, all of the elements noted above are assembled, the legs are passed through the apertures, and the end of each leg is formed into a nub. Alternately, one end may be formed into a nub prior to placement through the aperture, with the second end being formed into a nub after assembly of all the elements.

The number of connectors and staples are typically dictated by the size and structure of a particular stent; it is intended that the invention should not be limited thereby.

The above staple aperture design or connector assembly has many advantages for attaching a stent to a graft. Because the legs of the staple are folded around and imbedded within a pocket or the like, any risk of puncturing an inflation balloon is minimized. In addition, the structural integrity of the prosthesis is increased because staples more securely attach the graft material to the stent, as compared to prior art designs which use suture or adhesives to attach the graft to the stent.

Staples 90 and 120 (in Figures 3 and 7) may be made from any number of materials known in the art, including tantalum alloys, platinum alloys or stainless steel, such as a grade of type 316 stainless steel. The staples may take on other configurations and shapes, and can be coated for lubricity purposes, wear resistance and for the prevention of corrosion. Essentially, the coating may be used for increased durability . The staples may be formed from a radiopaque material to identify the location of the staple, and to act as a marker to identify the location of a portion of the prosthesis. Using a different number of radiopaque staples on a distal end of a stent as compared to a proximal end further assists in identifying the position of the prosthesis.

### METHODS

A method in accordance with the present invention includes delivering and positioning a system or component of a system in a fluid conduit, such as an aorta. The components described above permit intraluminal delivery into an aorta. This is accomplished by percutaneously inserting the prostheses into the same or different arteries, e.g., a femoral artery, and navigating them to the site of the aneurysm. This type of procedure is similar to delivery of angioplasty catheters and guiding catheters into the human vasculature. Upon proper positioning, the system components may be deployed either through a radially, outwardly extending force, e.g., expanding a balloon, or, if a self-expanding stent, by releasing the stent from a constraint. Once fully deployed, at least one passageway is formed bypassing the aneurysm. As shown in Figure 1, it may be desirable to form two fluid flow paths bypassing the aneurysm, each fluid flow path extending into a separate downstream artery.

In preferred embodiments of the invention, the first prosthesis is a sealing prosthesis, even more preferably, a first prosthesis that expands automatically against the wall of the artery. The method also includes delivering and positioning at least one second prosthesis. In preferred embodiments of the invention, the second prosthesis is a bypass conduit for extending through an aneurysm. The second prosthesis is typically positioned within the first prosthesis, preferably matingly engaging a manifold in a distal portion of the prosthesis. In most preferred embodiments of the invention, the hole is slightly smaller in diameter than the expanded diameter of the second prosthesis, thus sealingly engaging the second prosthesis in the first prosthesis. The sealed configuration of the second prosthesis within the first prosthesis forms a fluid pathway through the assembly or system, thereby bypassing the aneurysm.

For embodiments of the invention as illustrated in Figure 2, the method may further include delivering and positioning at least one third prosthesis. In preferred embodiments of the invention, the third prosthesis is a bypass conduit for extending from the proximal end of the system into a cross artery. The third prosthesis is typically positioned within the first prosthesis, preferably into and through a hole in the second manifold. In most preferred embodiments of the invention, the hole is slightly smaller in diameter than the expanded diameter of the third prosthesis, thus sealingly engaging the third prosthesis in the first prosthesis. The sealed configuration of the third prosthesis within the first prosthesis forms a fluid pathway or channel through a portion of the assembly or system into an artery or position upstream of the aneurysm.

Figures 1 and 2 generally show how the system of the present invention may be deployed *in vivo*. One skilled in the art will readily recognize that a typical delivery device, such as a catheter, includes a guidewire or the like that passes through an aperture in the cover of the first prosthesis, and a collar or the like that releasably engages at least one anchor on the prosthesis. Once the anchors are released from the collar, the first prosthesis can expand, preferably automatically. The portion of the delivery device containing the collar can then be removed from the artery, typically leaving the guidewire in place, i.e., still positioned in an aperture of the first prosthesis cover. The guidewire can then be used to guide another prosthesis or prostheses into position.

In some embodiments of the invention, the collar of the delivery device, engaged to the prosthesis, may be positioned within a sheath or the like until the prosthesis is delivered. In preferred embodiments of the invention, a portion of the prosthesis may be partially deployed and/or positioned. Once it is determined that the prosthesis is in its proper position, the collar can be pushed out of the sheath, thereby releasing the anchors from the collar. If the prosthesis is a self-expanding prosthesis, release of the flanges will allow the prosthesis to deploy automatically. If the prosthesis is not self-expanding, a deflated balloon or the like may be delivered to the interior of the prosthesis using the guidewire. When the balloon is inflated, it will expand the prosthesis into its fully deployed position, i.e., fully expanded radially.

As is evident to one skilled in the art, precisely placing a component(s) of the system may be critical. The physician must have precise placement of the components to ensure adequate repair of the aneurysm. The present invention allows the physician to fully deploy a component within the body without fully releasing the entire component from the delivery device. The anchors releasably interlock with complementary structures, such as grooves, on the delivery device, and, if the physician decides that the placement of the component is incorrect, the outer member of the delivery device may be moved relative to an inner member, thereby resulting in the prosthesis being retrieved or retracted within the delivery device. The extended legs and anchors allow the physician to temporarily position the prosthesis before full deployment. Once the physician is satisfied with a prosthesis' position, the legs may be released from their engagement with the delivery device.

After proper delivery, first prosthesis 10 and second prostheses 11a,b should appear as they do in Figures 1 or 2. First prosthesis 10 is firmly secured within an arterial section upstream of an aneurysm, and may or may not extend into one or more arteries. For example, the first prosthesis or a portion thereof may be positioned upstream of an arterial junction or downstream of the junction. Second prostheses 11a and 11b provide a fluid flow path that extends through the aneurysm, anchoring in an artery downstream of the aneurysm. Third prostheses 11c and 11d provide a fluid flow path that extends into cross arteries upstream of the aneurysm, anchoring in a downstream portion of the cross artery.

In one exemplary embodiment of the invention, a proximal portion of the first prosthesis is positioned upstream of the renal arteries, a distal portion of the first prosthesis is positioned downstream of the renal arteries, for example, in the infrarenal neck region, and an intermediate portion of the first prosthesis is positioned across the junction between the renal arteries and the abdominal aorta. The outward force of the second prostheses 11a,b on the first prosthesis 10 helps to secure the device within the body. The distal ends of the second prosthesis may be firmly attached to the iliac arteries 1 and 2. Thereafter blood will flow from the abdominal aorta, through an exemplary system of the present invention comprising a first prosthesis and two second prostheses 11(a) and 11(b), and into iliac arteries 1 and 2, thereby bypassing the aneurysm 100. In this embodiment of the invention, fluid may freely pass through an intermediate portion of the system into renal arteries 3 and 4.

In an alternate exemplary embodiment, the system is further configured with third prostheses 11c and 11d, and fluid is directed through the prostheses into renal arteries 3 and 4.

In accordance with the present invention, a system and method for bypassing an aneurysm may establish one, and possible multiple, fluid flow paths through the system. When the system is placed in an artery upstream of a junction with one or more other arteries, the system permits fluid, such as blood, to flow through the proximal end of the system, and a portion of the blood may flow out of the system into one of the cross arteries. Another portion of the fluid will continue within the system, bypassing the aneurysm and out of the system into one or more downstream arteries. A method of the present invention therefore includes establishing one or more fluid flow paths. In a preferred embodiment of the invention, the method includes establishing a first fluid flow path through the system, wherein the first fluid flow path bypasses the aneurysm. The method may further include establishing at least one second fluid flow path, wherein the second fluid flow path passes through a portion of the system, and passes out of an intermediate portion of the system into an artery or arteries.

In preferred embodiments of the invention, the system is used to bypass an abdominal aorta aneurysm (AAA). A method for treating or bypassing an abdominal aorta includes delivering, preferably percutaneously, a first prosthesis or one of its components (e.g., the gasket member may be delivered separately, if desired). The components of the system are typically delivered through one of the femoral arteries and deployed within the infrarenal neck, between an abdominal aortic aneurysm and the renal arteries of a patient. Once the first prosthesis is properly positioned or re-positioned, the flanges or anchors are fully released from the delivery device. The delivery device for the first prosthesis may then be removed, without removing the guidewire, and another guidewire may be inserted through the other femoral artery and into first prosthesis. If the second guidewire is on the wrong side of the interior of first prosthesis, it will contact the occlusive member and be prevented from easily advancing. The physician may then properly reposition the guidewire.

Thereafter each delivery apparatus, each containing a sheathed second prosthesis, is inserted into femoral arteries 1 and 2 by sliding them over the guide wires; each of the two second prostheses are then positioned in the first prosthesis. Thereafter, the second prostheses may be either separately or simultaneously deployed.

It is important to note that even though self-expanding stents are utilized, balloons may be utilized for tacking them into position if necessary.

### ALTERNATE CONFIGURATIONS

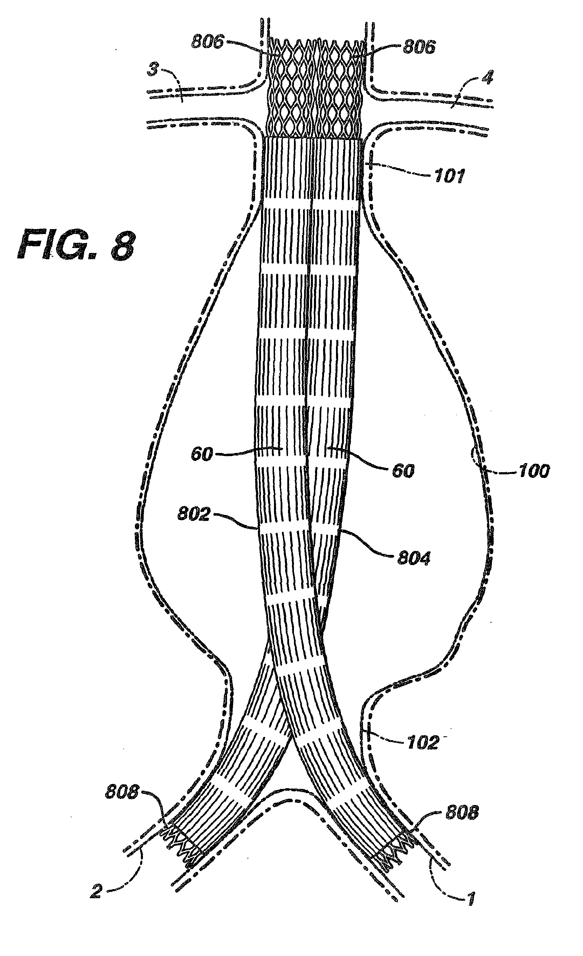

Referring to Figure 8, there is illustrated an alternate exemplary embodiment of the modular aneurysm repair system of the present invention. In this exemplary embodiment two stent-grafts 802, 804 are anchored upstream of the aneurysm 100 and across at least a portion of the renal arteries 3, 4. The stent-grafts 802, 804 may be similar in design to the second prostheses described above. In this exemplary embodiment, however, a proximal section 806 of each stent-graft 802, 804 is uncovered by graft material 60. The bare stent proximal section 806 is long enough to provide sufficient anchoring in diseased or angulated sections of artery while allowing substantially unimpeded blood flood into cross arteries such as the renal arteries 3, 4. Accordingly, even if the section of the aorta in which the stent-grafts 802, 804 is highly angulated, the bare stent proximal sections 806 secure the stent-grafts 802, 804 in position.

The stent-grafts 802, 804 may also comprise a distal section 808 of uncovered stent. The bare stent distal section 808 is long enough to provide sufficient anchoring in arteries downstream of the aneurysm 100, for example, the iliac arteries 1, 2 while allowing substantially unimpeded blood flow into branch arteries such as the internal iliac arteries.

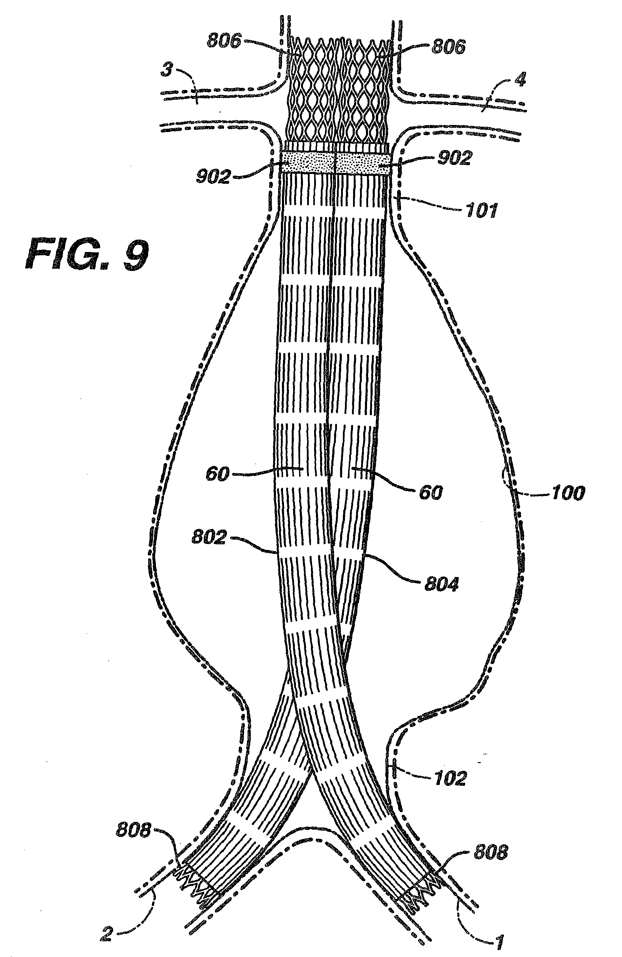

The graft material 60 may comprise any of the materials suggested above that are substantially impervious to blood flow. As illustrated, the graft material 60 of each stent-graft 802, 804 starts a sufficient distance above the aneurysm 100 to prevent any endo-leaks. In another exemplary embodiment, for example, as illustrated in Figure 9, a gasket material 902 may be positioned around each of the stent-grafts 802, 804 adjacent to the base stent proximal section 806 to prevent endo-leaks.

The gasket material or sealing ring 902 may be configured in any suitable manner and may comprise any suitable material. Exemplary materials are composed of a biodurable and biocompatible material, including but are not limited to open cell foam materials and closed cell foam materials. Exemplary materials include polyurethane, polyethylene, polytetrafluoroethylene, and other various polymer materials, preferably woven or knitted, that provide a flexible structure, such as polyester (such as that sold under the trade mark Dacron). Highly compressible foams are particularly preferred, preferably to keep the crimped profile low for better delivery. The gasket material or foam is preferably substantially impervious to blood when in a compressed state.

## Claims

**1.** A system for bypassing an aneurysm comprising a first prosthesis and at least one second prosthesis communicating with the first prosthesis, said first prosthesis comprising a conduit defining a fluid flow path; wherein said second prosthesis is configured to provide a fluid flow path through the aneurysm.

**2.** The system of claim 1 wherein the first prosthesis comprises a stent and a graft material communicating with the stent.

**3.** The system of claim 2 wherein said stent and graft material define a fluid flow path through the prosthesis.

**4.** The system of claim 1 wherein said first prosthesis further comprises at least one gasket configured to receive at least one second prosthesis.

**5.** The system of claim 4 wherein said gasket is configured to receive two second prosthesis.

**6.** The system of claim 1 wherein the second prosthesis comprises a stent and a graft material communicating with the stent.

**7.** The system of claim 6 wherein said stent and graft material define a fluid flow path through the prosthesis.

**8.** The system of claim 7 wherein the fluid flow path is a channel that bypasses the aneurysm.

**9.** A system for bypassing an aneurysm comprising a first prosthesis defining a first fluid path, at least two second prosthesis communicating with the first prosthesis, said first prosthesis comprising a proximal end configured to engage a section of artery upstream of an aneurysm; said second prosthesis being configured to bypass the aneurysm and anchor in an artery downstream of the aneurysm.

**13.** The system of claim 1 wherein the first prosthesis is adapted to conform to the shape of the artery.

**14.** The system of claim 13 wherein adapted to conform to the shape of the artery comprises a first prosthesis having a flexible intermediate portion.

**15.** The system of claim 1 wherein the first prosthesis further comprises a manifold configured to receive at least one second prosthesis.

**16.** The system of claim 15 wherein said manifold is configured to split the fluid flow path into at least two fluid flow paths.

**17.** A system for bypassing an aneurysm comprising:
a first stent-graft having a bare stent proximal section, the first stent-graft being positioned such that the bare stent proximal section allows blood flow into cross-arteries; and
a second stent-graft having a bare stent proximal section, the second stent graft being positioned such that the bare stent proximal section allows blood flow with cross-arteries.

**18.** A system for bypassing an aneurysm comprising:
a first stent-graft having a first bare stent proximal section and a first sealing gasket adjacent the first bare stent proximal section; and
a second stent-graft having a second bare stent proximal section and a second sealing gasket adjacent the second bare stent proximal section.
